(19) Europäisches Patentamt
European Patent Office
Office européen des brevets

(11) Veröffentlichungsnummer: **0 392 332 A2**

# (12) EUROPÄISCHE PATENTANMELDUNG

(21) Anmeldenummer: **90106444.4**

(51) Int. Cl.$^5$: **G01N 33/94**

(22) Anmeldetag: **04.04.90**

(30) Priorität: **12.04.89 US 337040**

(43) Veröffentlichungstag der Anmeldung:
**17.10.90 Patentblatt 90/42**

(84) Benannte Vertragsstaaten:
**AT BE CH DE DK ES FR GB IT LI NL SE**

(71) Anmelder: **F. HOFFMANN-LA ROCHE AG**
**Postfach 3255**
**CH-4002 Basel(CH)**

(72) Erfinder: **Kaufman, Richard Allen**
**744 Joralemon Street**
**Belleville N.J. 07109(US)**
Erfinder: **Schwenzer, Kathryn Sarah**
**151 Forest Avenue**
**Verona, N.J. 07044(US)**
Erfinder: **Wu, Lynne Ling-Tzyy**
**34 Mt. Herman Way**
**West Caldwell, N.J. 07006(US)**

(74) Vertreter: **Kloter, Rudolf et al**
**Postfach 3255 Grenzacherstrasse 124**
**CH-4002 Basel(CH)**

## (54) Fluoreszenzpolarisationsimmunoassay und Reagentien dafür.

(57) Ein Reagens zur Verwendung beim Fluoreszenzpolarisationsimmunoassay für 11-nor-Δ9-Tetrahydrocannabinol-9-carbonsäure (ein wichtiger Metabolit des Rauschgiftes Marihuana) enthält neben einem Antikörper für diesen Metaboliten und einem wässrigen Puffer ein Freisetzungsmittel. Dieses Freisetzungsmittel bindet an und blockiert unspezifische Bindungsstellen des im Urin vorhandenen Humanserumalbumins und von anderen Proteinen im Urin und ermöglicht dadurch eine zuverlässigere Erfassung des so "freigesetzten" Metaboliten. Auch werden Varianten eines unter Verwendung der Freisetzungsmittel durchgeführten Verfahrens zur Erfassung des Metaboliten beschrieben.

EP 0 392 332 A2

## Fluoreszenzpolarisationsimmunoassay und Reagentien dafür

Bei der Bestimmung des Rauschgifts Marihuana im Urin stellt üblicherweise ein Wert von 100 Nanogramm (ng) seines Metaboliten 11-nor-Δ9-Tetrahydrocannabinol-9-carbonsäure/ml Urin den Schwellenwert für ein positives Ergebnis dar, wie dies in den NIDA (National Institute of Drug Abuse)-Guidelines 1988 festgelegt ist. Dies bedeutet, dass ein Wert von mehr als 100 ng/ml Urin die Gegenwart von Marihuana (positives Ergebnis), hingegen ein Wert von weniger als 100 ng/ml Urin dessen Abwesenheit (negatives Ergebnis) anzeigt. Zur Vermeidung von falsch negativen Ergebnissen ist es deshalb notwendig, den gesamten Metaboliten 11-nor-Δ9-Tetrahydrocinnabinol-9-carbonsäure (Δ9-THC-9-COOH) in verschiedenen Typen von Urinproben erfassen zu können. Da in einigen Urinproben hohe Konzentrationen von Proteinen, wie Humanserumalbumin, vorhanden sind, bindet die Δ9-THC-9-COOH in solchen Urinproben unspezifisch an Bindungsstellen dieses Humanserumalbumins und von anderen Proteinen, was eine quantitative Bildung des Antikörper-Komplexes und demzufolge einen quantitativen Assay für die vorhandene Δ9-THC-9-COOH verhindert. Dies kann selbstverständlich bei gewissen Urinproben zu falsch negativen Ergebnissen führen.

Aufgabe der Erfindung ist es, einen quantitativen Fluoreszenzpolarisationsimmunoassay für 11-nor-Δ9-Tetrahydrocannabinol-9-carbonsäure im Urin zu ermöglichen, bei dem falsch negative Ergebnisse ausgeschlossen sind, sowie einen qualitativen Fluoreszenzpolarisationsimmunoassay für 11-nor-Δ9-Tetrahydrocannabinol-9-carbonsäure im Urin bereitzustellen.

Diese Aufgabe wird erfindungsgemäss gelöst durch die Verwendung eines sogenannten Freisetzungs- oder Blockierungsmittels (im folgenden lediglich als Freisetzungsmittel bezeichnet). Bei diesem Freisetzungsmittel handelt es sich um eine chemische Verbindung, die bei Zusatz zu einer auf Marihuana zu prüfenden Urinprobe bzw. Antikörperreagens--Urin-Mischung mit dem "Analyten" oder "Ligand" (also Metaboliten Δ9-THC-9-COOH) um unspezifische Bindungsstellen des Humanserumalbumins und von anderen Proteinen im Urin konkurriert. Dieses Freisetzungsmittel wird in einer überschüssigen Menge zugegeben, damit es an sämtliche Stellen einer klinischen Urinprobe mit extrem hohem Proteingehalt binden kann. Bei einer solchen Menge konkurriert das Freisetzungsmittel erfolgreich mit dem Analyten um die unspezifischen Bindungsstellen, und zwar mit dem Ergebnis, dass die Bindungsstellen durch das Freisetzungsmittel "blockiert" werden und der Analyt von Interesse bindungsunfähig wird. Demzufolge bleibt der ganze (oder nahezu der ganze) Analyt in Lösung und kann erfasst werden. Er wird also durch die Aktion des Freisetzungsmittels zur Erfassung "freigesetzt". Bekannt ist die Verwendung von Freisetzungs- oder Blockierungsmitteln in Serumassays, nicht aber in Urinassays.

Gegenstand der vorliegenden Erfindung sind ein Reagens zur Verwendung beim Fluoreszenzpolarisationsimmunoassay für 11-nor-Δ9-Tetrahydrocannabinol-9-carbonsäure, eine mit einem Freisetzungsmittel behandelte Urinprobe zur Unter suchung in einem solchen Fluoreszenzpolarisationsimmunoassay sowie Verfahren zur quantitativen oder qualitativen Erfassung von 11-nor-Δ9-Tetrahydrocannabinol-9-carbonsäure in einem Fluoreszenzpolarisationsimmunoassay.

Das erfindungsgemässe Reagens zur Verwendung beim Fluoreszenzpolarisationsimmunoassay für 11-nor-Δ9-Tetrahydrocannabinol-9-carbonsäure ist dadurch gekennzeichnet, dass es einen Antikörper für 11-nor-Δ9-Tetrahydrocannabinol-9-carbonsäure; ein Freisetzungsmittel, das aus 1-Anilinonaphthalin-8-sulfonsäure (ANS), 1,1′-bis(4-Anilino)naphthalin-5,5′-disulfonsäure (bis-ANS), 2-Anilinonaphthalin-6-sulfonsäure (2,6-ANS), Lithiumdodecylsulfat (LDS), Natriumdodecylsulfat (SDS), Ammoniumdodecylsulfat, Cholsäure, Benzoesäure, Salicylsäure, 5-Chlorsalicylsäure (5-Chlor-2-hydroxybenzoesäure), 5-Jodsalicylsäure (2-Hydroxy-5-jodbenzoesäure), 5-Methoxysalicylsäure (2-Hydroxy-5-methoxybenzoesäure), 5-Sulfosalicylsäure (2-Hydroxy-5-sulfobenzoesäure), Witconate P 10-59 und Gemischen dieser Substanzen ausgewählt ist; sowie einen wässrigen Puffer mit einem pH-Bereich von 7,0 bis 8,5 enthält.

Bei dem im obigen Reagens als Freisetzungsmittel verwendbaren "Witconate P 10-59" handelt es sich um ein oberflächenaktives Mittel der Witco Corporation, 520 Madison Avenue, NY 10022, USA.

Der im obigen Reagens verwendete Antikörper (woanders auch als "Antiserum" bezeichnet) ist ein Protein, das in herkömmlicher Weise erzeugt werden kann, und zwar dadurch, dass man ein Hapten, also im jetzigen Fall den Metaboliten ("Analyten", "Ligand") 11-nor-Δ9-Tetrahydrocannabinol-9-carbonsäure, an ein Trägerprotein bindet und das resultierende Bindungsprodukt zur Erzeugung des Antikörpers einem Wirtstier, z.B. einer Ziege, injiziert. Anschliessend wird der so erzeugte Antikörper zu Δ9-THC-9-COOH auf konventionelle Weise isoliert. Dieser Antikörper weist die "spezifischen" Bindungsstellen auf, an welche Δ9-THC-9-COOH bindet, im Gegensatz zu den oben bereits erwähnten konkurrierenden "unspezifischen" Bindungsstellen in Humanserumalbumin und anderen in Urin vorhandenen Proteinen. Man bezeichnet das Produkt der Kupplung des Metaboliten mit dem Antikörper als einen Ligand-Antikörper-Komplex.

Die erfindungsgemäss verwendeten Freisetzungsmittel sind hydrophil und im erfindungsgemässen

Reagens bzw. Fluoreszenzpolarisationsimmunoassay in einer solchen Menge vorhanden, die eine möglichst vollständige Erfassung der in der zu untersuchenden Urinprobe vorhandenen Δ9-THC-9-COOH erlaubt, allerdings nicht in einer Menge, bei der die Wirksamkeit des Antikörpers wesentlich beeinträchtigt wird. Demgemäss soll das jeweilige Freisetzungsmittel eine hohe Affinität für die unspezifischen Bindungsstellen haben. Vorzugsweise übersteigt die Menge an Freisetzungsmittel in Mol/l die Menge (Mol/l) an Proteinen im Reagens. Klarerweise hängt die ideale Menge an Freisetzungsmittel für den fraglichen Assay u.a. vom Volumen der Urinprobe, von deren Alter, von der Menge Antikörper und von der Menge des markierten Analyten ab. Die bevorzugte Menge an Freisetzungsmittel ist die Menge, die eine prozentuale Erfassung von mindestens 85%, insbesondere 95-100%, der in der Urinprobe vorhandenen 11-nor-Δ9-Tetrahydrocannabinol-9-carbonsäure ermöglicht.

Die bevorzugten Freisetzungsmittel sind 1-Anilinonaphthalin-8-sulfonsäure (ANS), 1,1′-bis(4-Anilino)-naphthalin-5,5′-disulfonsäure (bis-ANS), Lithiumdodecylsulfat (LDS) und Gemische davon, insbesondere bis-ANS allein.

Der im erfindungsgemässen Reagens vorhandene Puffer kann konventionell sein, wie diese in handelsüblichen Antikörper-Reagentien aus Antikörpern und Puffern vorkommen. Solche Puffer sind dazu fähig, den pH-Wert des Reagens im Bereich von 7 bis 8,5 beizuhalten, und weisen einen pKa-Wert von 7 bis 8,5 auf. Es eignet sich als wässriger Puffer insbesondere 0,1M Phosphat-Puffer. Weitere geeignete Puffer sind Tris [Tris(hydroxymethyl)aminomethan], Bis-Tris [N,N-Di-(2-hydroxyäthyl)-tri(hydroxymethyl)methylamin] und Phosphat-gepuffertes Salzwasser.

Das erfindungsgemässe Reagens enthält vorzugsweise auch noch Rinder-gamma-globulin und Natriumazid, wobei die Menge des erstgenannten Zusatzes vorteilhaft ca. 0,01 w/v% beträgt und die Menge des Natriumazids ca. 0,2 w/v% beträgt. Unter dem hier und im folgenden verwendeten Ausdruck "w/v%" ist ein prozentuales Verhältnis Gewicht: Volumen zu verstehen. Beispielsweise bedeutet "1,0 w/v% Salicylsäure" eine Konzentration von 1 g Salicylsäure/100 ml Lösung, und "0,005 w/v% ANS" 0,005 g ANS/100 ml Lösung.

Eine besonders bevorzugte Ausführungsform des erfindungsgemässen Reagens enthält aus einer Ziege gewonnenes Antiserum, ca. 0,1M Phosphat-Puffer mit einem pH-Wert von ca. 8, ca. 0,01 w/v% Rinder-gamma-globulin, ca. 0,2 w/v% Natriumazid sowie Freisetzungsmittel, wobei das Freisetzungsmittel selber besonders bevorzugt 1,1′-bis(4-Anilino)naphthalin-5,5′-disulfonsäure bei einer Konzentration von ca. 0,0005 w/v% ist.

Konventionelle Antikörper-Reagentien werden dadurch hergestellt, dass man den Antikörper, dessen Erzeugung oben beschrieben ist, mit wässrigem Puffer, wie dieser ebenfalls oben beschrieben ist, vereinigt. Das erfindungsgemässe Reagens kann dadurch hergestellt werden, dass man beispielsweise einem derartigen konventionellen Antikörper-Reagens zur Verwendung beim Fluoreszenzpolarisationsimmunoassay eines oder mehrere der oben angegebenen Freisetzungsmittel zufügt.

Der erfindungsgemässe Fluoreszenzpolarisationsimmunoassay kann nach verschiedenen Verfahren erfolgen.

Das eine erfindungsgemässe Verfahren zur quantitativen oder qualitativen Erfassung von 11-nor-Δ9-Tetrahydrocannabinol-9-carbonsäure in einem Fluoreszenzpolarisationsimmunoassay ist dadurch gekennzeichnet, dass man einem Antikörper-Reagens zur Verwendung beim Fluoreszenzpolarisationsimmunoassay für 11-nor-Δ9-Tetrahydrocannabinol-9-carbonsäure eines oder mehrere der oben angegebenen Freisetzungsmittel zufügt.

Anschliessend kann man den Fluoreszenzpolarisationsimmunoassay so durchführen, dass man dem auf obige Weise behandelten Antikörper-Reagens (das dem erfindungsgemässen Reagens entspricht) die zu untersuchende Urinprobe zugibt, das Gemisch Hintergrundfluoreszenzintensitätsmessungen unterwirft, einen markierten Analyten zugibt, und die Fluoreszenzintensitätswerte des Gemisches misst. Aus den Messungen kann man die Fluoreszenzpolarisation berechnen sowie die Konzentration des Analyten unter Zuhilfenahme einer Standardkalibrierungskurve bestimmen. Eine Konzentration von grösser als 100 ng/ml Urin gilt in diesem Assay als ein positives Ergebnis (Anwesenheit des Analyten 11-nor-Δ9-Tetrahydrocannabinol-9-carbonsäure im Urin), hingegen eine Konzentration von weniger als 100 ng/ml Urin als ein negatives Ergebnis (Abwesenheit des Analyten im Urin).

Unter dem oben und im folgenden verwendeten Ausdruck "markierten Analyten" versteht man den Analyten, an den eine fluoreszierende Verbindung, wie beispielsweise Fluoresceinisothiocyanat der Formel

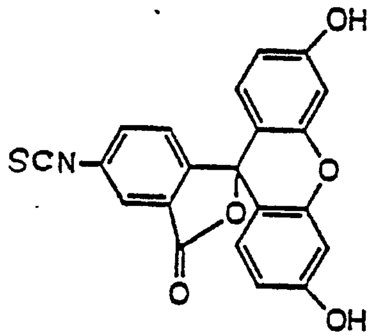

gekoppelt ist, und die folgende Formel aufweist:

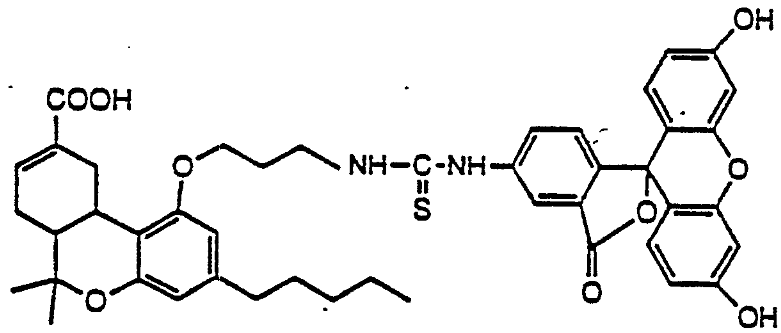

I

(siehe in diesem Zusammenhang die Europäische Patentpublikation Nr. 276.732).

Der im Urin vorhandene Analyt (Δ9-THC-9-COOH) konkurriert im Polarisationsimmunverfahren mit dem markierten Analyten um die freien Bindungsstellen des Antikörpers.

Die obenerwähnte Standardkalibrierungskurve wird von Fluoreszenzpolarisationsintensitätsmessungen an wässrigen Analyt-Lösungen bekannter Konzentrationen erstellt.

Ein qualitativer Fluoreszenzpolarisationsimmunoassay bezweckt lediglich die Feststellung der An- oder Abwesenheit des Rauschgift-Metaboliten in der Urinprobe, nicht das Eruieren dessen absoluter Menge. Das letztere wird durch einen quantitativen Fluoreszenzpolarisationsimmunoassay bestimmt.

Als Alternative zum oben beschriebenen Verfahren kann man zunächst einmal der zu untersuchenden Urinprobe eines oder mehrere der oben angegebenen Freisetzungsmittel zufügen. Demgemäss ist das weitere erfindungsgemässe Verfahren zur quantitativen oder qualitativen Erfassung von 11-nor-Δ9-Tetrahydrocannabinol-9-carbonsäure in einem Fluoeszenzpolarisationsimmunoassay dadurch gekennzeichnet, dass man der zu untersuchenden Urinprobe eines oder mehrere der oben angegebenen Freisetzungsmittel zufügt. Wie bereits oben erwähnt, wird gemäss diesem Verfahren das Freisetzungsmittel in einer überschüssigen Menge zugegeben, so dass es an sämtliche Stellen des in der Urinprobe vorhandenen Humanserumalbumins und von anderen vorhandenen Proteinen binden kann, um die allfällig ebenfalls im Urin vorhandene 11-nor-Δ9-Tetrahydrocannabinol-9-carbonsäure "freizusetzen", aber nicht in einer solchen Menge, bei der die Wirksamkeit des Antikörpers beeinträchtigt würde. Die bevorzugte Menge an Freisetzungsmittel ist diejenige, die eine prozentuale Erfassung von mindestens 85%, insbesondere 95-100%, der vorhandenen 11-nor-Δ9-Tetrahydrocannabinol-9-carbonsäure ermöglicht.

Nach Hinzufügen des Freisetzungsmittels zur Urinprobe kann man den Fluoreszenzpolarisationsimmunoassay so durchführen, dass man die behandelte Urinprobe einem Antikörper-Reagens, das sich vom erfindungsgemässen Reagens durch die Abwesenheit von Freisetzungsmittel unterscheidet, zugibt, das Gemisch Hintergrundfluoreszenzintensitätsmessungen unterwirft, markierten Analyten zugibt und die Fluoreszenzintensitätswerte des Gemisches misst. Wie im Fall des ersten oben beschriebenen Verfahrens kann man aus den Messungen die Fluoreszenzpolarisation berechnen sowie die Konzentration des Analyten bestimmen, und das Ergebnis wird in gleicher Weise bewertet. Als Alternative kann man den markierten Analyten unmittelbar nach Vereinigung der Urinprobe und des Freisetzungsmittels der behandelten Urinprobe zufügen und hinterher zum Antikörper-Reagens geben. Anschliessend werden die Fluoreszenzpolarisationsmessungen durchgeführt, die Konzentration des Analyten bestimmt und das Ergebnis bewertet.

Zur Messung der Fluoreszenzpolarisation eignet sich das COBAS BIO FP®-Gerät.

Die oben beschriebenen mit Freisetzungsmittel behandelten Urinproben, die gegebenenfalls mit Wasser verdünnt werden können, bilden einen weiteren Gegenstand der vorliegenden Erfindung. Die erfindungsgemässe wässrige Lösung ist gekennzeichnet durch einen Gehalt an Urin sowie einem der oben angegebenen Freisetzungsmittel oder einem Gemisch davon.

Die nachfolgenden Beispiele dienen zur näheren Erläuterung der Erfindung:

## Beispiel 1

In diesem Beispiel wurden Urinproben verwendet, denen jeweils 0,05 w/v% Humanserumalbumin (eine Menge, die in normalem Urin als erhöht gilt) sowie 100 ng/ml 11-nor-Δ9-Tetrahydrocannabinol-9-carbonsäure zugegeben wurden.

Die mit markiertem Analyten (3 x $10^{-7}$ M/l) versehene Puffer-Lösung bestand aus 0,1M ACES ($H_2NCOCH_2NHCH_2CH_2SO_3H$), Puffer pH 8, 0,01 w/v% Rinder-gamma-globulin sowie 0,1 w/v% Natriumazid.

Das verwendete Verfahren war dasjenige, bei dem man dem Antikörper-Reagens ein Freisetzungsmittel zufügt.

Vor jeder Messung wurde das Antiserum verdünnt. Beispielsweise wurde im Falle der Verwendung des Freisetzungsmittels Salicylsäure bei einer Konzentration von 0,2 w/v% (siehe Tabelle 1) 1 Volumen Antiserum in Antikörper-Puffer gelöst, um zu einem 3600 mal-höheren Endvolumen zu gelangen.

Die Messung der Fluoreszenzpolarisation erfolgte unter Verwendung des COBAS BIO FP®-Gerats.

Die Ergebnisse der Untersuchung der verschiedenen Freisetzungsmittel im diesbezüglichen erfindungsgemässen Verfahren sind in der nachstehenden Tabelle 1 zusammengefasst:

Tabelle 1

| Freisetzungsmittel mit Antikörper-Reagens vorgemischt; Erfassung von Δ9-THC-9-COOH im Urin und mP-Spannen | | | | |
|---|---|---|---|---|
| Freisetzungsmittel, w/v% | mP-Spanne* 0-50 ng/ml | mP-Spanne* 0-100 ng/ml | Probevolumen in µl | % Erfassung von 9-THC-9-COOH |
| Keines | 46 | 119 | 14 | 49 |
| 0,2 Salicylsäure | 33 | 103 | 14 | 100 |
| 0,0005 LDS | 40 | 108 | 14 | 100 |
| 0,01 ANS | 28 | 106 | 14 | 90 |
| 0,0005 bis-ANS | 39 | 116 | 14 | 100 |

* mP-Spanne ist eine Messung des Unterschieds zwischen der Netto-Fluoreszenzmillipolarisation an der Stelle, an der die maximale und die minimale Menge Tracer an den Antikörper gebunden ist.

Aus den Ergebnissen ist ersichtlich, dass alle vier geprüften Freisetzungsmittel, nämlich Salicylsäure, Lithiumdodecylsulfat, 1-Anilinonaphthalin-8-sulfonsäure und 1,1′-bis(4-Anilino)naphthalin-5,5′-disulfonsäure, als sehr wirksam im Fluoreszenzpolarisationsimmunoassay gelten. Im Falle, wo kein Freisetzungsmittel verwendet wurde, wurde weniger als die Hälfte der Δ9-THC-9-COOH erfasst.

## Beispiel 2

In diesem Beispiel wurden Urinproben verwendet, zu denen nicht nur 0,05 w/v% Humanserumalbumin und 100 ng/ml Δ9-THC-9-COOH sondern auch noch Freisetzungsmittel gegeben wurden. Im Falle der Verwendung von 0,005 w/v% bis-ANS als Freisetzungsmittel wurden ca. 40 µl Urin und 120 µl 0,005 w/v% bis-ANS zusammengemischt. Anschliessend wurden 42 µl des Gemisches [äquivalent zu 14 µl Urin (Probevolumen)] dem Antikörper-Reagens zugefügt und die Hintergrundfluoreszenzintensitätsmessungen

durchgeführt.

Nach Zugabe des markierten Analyten wurden Fluoreszenzpolarisationsintensitätswerte gemessen, und zwar wiederum unter Verwendung des COBAS BIO FP®-Geräts.

Die Ergebnisse der Untersuchung der verschiedenen Freisetzungsmittel im diesbezüglichen erfindungsgemässen Verfahren sind in der nachstehenden Tabelle 2 zusammengefasst:

Tabelle 2

| Freisetzungsmittel mit Urinprobe vorgemischt; Erfassung von Δ9-THC-9-COOH im Urin und mP-Spannen | | | | |
|---|---|---|---|---|
| Freisetzungsmittel, w/v% | mP-Spanne* 0-50 ng/ml | mP-Spanne* 0-100 ng/ml | Probevolumen in µl | % Erfassung von 9-THC-9-COOH |
| Keines | 46 | 119 | 14 | 49 |
| 0,005 LDS | 53 | 136 | 14 | 86 |
| 4 Salicylsäure | 44 | 117 | 14 | 96 |
| 4 Salicylsäure + 0,002 LDS | 41 | 125 | 14 | 96 |
| 4 Salicylsäure + 0,2 Cholsäure | 46 | 123 | 14 | 94 |
| 0,1 ANS | 46 | 116 | 13,3 | 92 |
| 0,005 bis-ANS | 49 | 142 | 14 | 100 |

* mP Spanne: siehe Beispiel 1.

Aus den Ergebnissen ist ersichtlich, dass alle sechs geprüften Freisetzungsmittel bzw. Freisetzungsmittelkombinationen, nämlich Lithiumdodecylsulfat, Salicylsäure, Salicylsäure-Lithiumdodecylsulfat-Gemisch, Salicylsäure-Cholsäure-Gemisch, 1-Anilinonaphthalin-8-sulfonsäure und 1,1′-bis-(4-Anilino)naphthalin-5,5′-disulfonsäure, bei den verwendeten Konzentrationen sehr wirksam sind. Unter diesen Freisetzungsmitteln befinden sich auch Kombinationen, nämlich Salicylsäure-LDS-Gemisch und Salicylsäure-Cholsäure-Gemisch. Auch in diesem Falle wurde festgestellt, dass weniger als die Hälfte der Δ9-THC-9-COOH erfasst wurde, wenn kein Freisetzungsmittel eingesetzt wurde. Auch die bevorzugten Konzentrationen der Freisetzungsmittel in den Urinproben (w/v%) wurden festgestellt:- Salicylsäure: 3-10, insbesondere ca. 4; LDS: 0,001-0,02, insbesondere ca. 0,005; ANS: 0,02-0,5: und bis-ANS: 0,003-0,1, insbesondere ca. 0,005.

## Beispiel 3

In diesem Beispiel wurden nochmals Urinproben verwendet, denen jeweils 0,05 w/v% Humanserumalbumin sowie 100 ng/ml 11-nor-Δ9-Tetrahydrocannabinol-9-carbonsäure zugegeben wurden.

Das verwendete Verfahren war dasjenige, bei dem man dem Antikörper-Reagens ein Freisetzungsmittel zufügt.

Als Puffer-Lösung wurde eine mit markiertem Analyten ergänzte, wie diese in Beispiel 1 beschrieben ist, verwendet.

Zur Messung der Fluoreszenzpolarisation wurde das COBAS BIO FP®-Gerät verwendet.

Die Ergebnisse der Untersuchung der verschiedenen Freisetzungsmittel im diesbezüglichen erfindungsgemässen Verfahren sind in der nachstehenden Tabelle 3 zusammengefasst:

Tabelle 3

| Freisetzungsmittel mit Antikörper-Reagens vorgemischt; Erfassung von Δ9-THC-9-COOH im Urin und mP-Spannen | | | | |
|---|---|---|---|---|
| Freisetzungsmittel, w/v% | mP-Spanne* 0-50 ng/ml | mP-Spanne* 0-100 ng/ml | Probevolumen in µl | % Erfassung von 9-THC-9-COOH |
| 0,2 Salicylsäure | 33 | 103 | 14 | 100 |
| 0,2 Salicylsäure + 0,0005 LDS | 40 | 108 | 14 | 100 |
| 0,2 Salicylsäure + 0,001 LDS | 42 | 72 | 10 | 100 |
| 0,2 Salicylsäure + 0,002 LDS | 26 | 65 | 10 | 100 |
| 0,001 ANS | 28 | 106 | 14 | 90 |
| 0,005 ANS | 41 | 113 | 14 | 85 |
| 0,0005 bis-ANS | 37 | 99 | 10 | 100 |
| 0,0005 bis-ANS | 39 | 116 | 14 | 100 |

* mP-Spanne: siehe Beispiel 1

Aus den Ergebnissen ist ersichtlich, dass sämtliche untersuchten Freisetzungsmittel bzw. Freisetzungsmittelkombinationen, nämlich Salicylsäure, Salicylsäure-Lithiumdodecylsulfat-Gemisch, 1-Anilinonaphthalin-8-sulfonsäure und 1,1′-bis(4-Anilino)naphthalin-5,5′-disulfonsäure, als sehr wirksam im Fluoreszenzpolarisationsimmunoassay gelten. Auch die bevorzugten Konzentrationen der Freisetzungsmittel in den Antikörper-Reagentien (w/v%) wurden festgestellt: -Salicylsäure: 0,1-0,4, insbesondere ca. 0,2; LDS: 0,0001-0,002, insbesondere ca. 0,0005; ca. 0,2 Salicylsäure mit ca. 0,002 LDS; ANS: 0,005-0,02, insbesondere ca. 0,01; und bis-ANS: 0,0001-0,002, insbesondere ca. 0,0005.

## Ansprüche

1. Reagens zur Verwendung beim Fluoreszenzpolarisationsimmunoassay für 11-nor-Δ9-Tetrahydrocannabinol-9-carbonsäure, dadurch gekennzeichnet, dass es einen Antikörper für 11-nor-Δ9-Tetrahydrocannabinolgcarbonsäure; ein Freisetzungsmittel, das aus 1-Anilinonaphthalin-8-sulfonsäure, 1,1′-bis(4-Anilino)naphthalin-5,5′-disulfonsäure, 2-Anilinonaphthalin-6-sulfonsäure, Lithiumdodecylsulfat, Natriumdodecylsulfat, Ammoniumdodecylsulfat, Cholsäure, Benzoesäure, Salicylsäure, 5-Chlorsalicylsäure, 5-Jodsalicylsäure, 5-Methoxysalicylsäure, 5-Sulfosalicylsäure, Witconate P 10-59 und Gemischen dieser Substanzen ausgewählt ist; sowie einen wässrigen Puffer mit einem pH-Bereich von 7,0 bis 8,5 enthält.

2. Reagens nach Anspruch 1, dadurch gekennzeichnet, dass es als Freisetzungsmittel 1-Anilinonaphthalin-8-sulfonsäure, 1,1′-bis(4-Anilino)naphthalin-5,5′-disulfonsäure oder Lithiumdodecylsulfat, oder ein Gemisch von zwei oder allen drei dieser Substanzen enthält.

3. Reagens nach Anspruch 2, dadurch gekennzeichnet, dass das Freisetzungsmittel 1,1-bis(4-Anilino)-naphthalin-5,5′-disulfonsäure ist.

4. Reagens nach einem der Ansprüche 1 bis 3, dadurch gekennzeichnet, dass das Freisetzungsmittel in einer solchen Menge vorhanden ist, die eine möglichst vollständige Erfassung der in der zu untersuchenden Urinprobe vorhandenen 11-nor-Δ9-Tetrahydrocannabinol-9-carbonsäure erlaubt, allerdings nicht in einer Menge, bei der die Wirksamkeit des Antikörpers wesentlich beeinträchtigt wird.

5. Reagens nach Anspruch 4, dadurch gekennzeichnet, dass die Menge an vorhandenem Freisetzungsmittel in Mol/l die Menge (Mol/l) an Proteinen im Reagens übersteigt.

6. Reagens nach einem der Ansprüche 1 bis 5, dadurch gekennzeichnet, dass die Menge an Freisetzungsmittel diejenige ist, die eine prozentuale Erfassung von mindestens 85% der in der Urinprobe vorhandenen 11-nor-Δ9-Tetrahydrocannabinol-9-carbonsäure ermöglicht.

7. Reagens nach Anspruch 6, dadurch gekennzeichnet, dass die Menge an Freisetzungsmittel eine prozentuale Erfassung von 95-100% der in der Urinprobe vorhandenen 11-nor-Δ9-Tetrahydrocannabinol-9-carbonsäure ermöglicht.

8. Reagens nach einem der Ansprüche 1 bis 7, dadurch gekennzeichnet, dass es zusätzlich zu den bereits genannten Bestandteilen auch noch Rinder-gamma-globulin und Natriumazid enthält.

9. Reagens nach Anspruch 8, dadurch gekennzeichnet, dass die Menge des Rinder-gamma-globulins ca. 0,01 w/v% und die Menge des Natriumazids ca. 0,2 w/v% betragen.

10. Reagens nach Anspruch 9, dadurch gekennzeichnet, dass es aus einer Ziege gewonnenes Antiserum, ca. 0,1M Phosphat-Puffer mit einem pH-Wert von ca. 8, ca. 0,01 w/v% Rinder-gamma-globulin, ca. 0,2 w/v% Natriumazid sowie Freisetzungsmittel enthält.

11. Reagens nach Anspruch 10, dadurch gekennzeichnet, dass als Freisetzungsmittel 1,1′-bis(4-Anilino)naphthalin-5,5′-disulfonsäure bei einer Konzentration von ca. 0,0005 w/v% verwendet wird.

12. Wässrige Lösung gekennzeichnet durch einen Gehalt an Urin sowie einem Freisetzungsmittel, das aus 1-Anilinonaphthalin-8-sulfonsäure, 1,1′-bis(4-Anilino)naphthalin-5,5′-disulfonsäure, 2-Anilinonaphthalin-6-sulfonsäure, Lithiumdodecylsulfat, Natriumdodecylsulfat, Ammoniumdodecylsulfat, Cholsäure, Benzoesäure, Salicylsäure, 5-Chlorsali cylsäure, 5-Jodsalicylsäure, 5-Methoxysalicylsäure, 5-Sulfosalicylsäure, Witconate P 10-59 und Gemischen dieser Substanzen ausgewählt ist.

13. Wässrige Lösung nach Anspruch 12, dadurch gekennzeichnet, dass sie als Freisetzungsmittel 1-Anilinonaphthalin-8-sulfonsäure, 1,1′-bis(4-Anilino)naphthalin-5,5′-disulfonsäure oder Lithiumdodecylsulfat, oder ein Gemisch von zwei oder allen drei dieser Substanzen enthält.

14. Wässrige Lösung nach Anspruch 13, dadurch gekennzeichnet, dass das Freisetzungsmittel 1,1′-bis-(4-Anilino)naphthalin-5,5′-disulfonsäure ist.

15. Wässrige Lösung nach einem der Ansprüche 12 bis 14, dadurch gekennzeichnet, dass die Menge an Freisetzungsmittel diejenige ist, die eine prozentuale Erfassung von mindestens 85% der im Urin vorhandenen 11-nor-Δ9-Tetrahydrocannabinol-9-carbonsäure ermöglicht.

16. Wässrige Lösung nach Anspruch 15, dadurch gekennzeichnet, dass die Menge an Freisetzungsmittel eine prozentuale Erfassung von 95-100% der im Urin vorhandenen 11-nor-Δ9-Tetrahydrocannabinol-9-carbonsäure ermöglicht.

17. Verfahren zur quantitativen oder qualitativen Erfassung von 11-nor-Δ9-Tetrahydrocannabinol-9-carbonsäure in einem Fluoreszenzpolarisationsimmunoassay, dadurch gekennzeichnet, dass man einem Antikörper-Reagens zur Verwendung beim Fluoreszenzpolarisationsimmunoassay für 11-nor-Δ9-Tetrahydrocannabinol-9-carbonsäure ein Freisetzungsmittel zufügt, das aus 1-Anilinonaphthalin-8-sulfonsäure, 1,1′-bis(4-Anilino)naphthalin-5,5′-disulfonsäure, 2-Anilinonaphthalin-6-sulfonsäure, Lithiumdodecylsulfat, Natriumdodecylsulfat, Ammoniumdodecylsulfat, Cholsäure, Benzoesäure, Salicylsäure, 5-Chlorsalicylsäure, 5-Jodsalicylsäure, 5-Methoxysalicylsäure, 5-Sulfosalicyl säure, Witconate P 10-59 und Gemischen dieser Substanzen ausgewählt ist.

18. Verfahren nach Anspruch 17, dadurch gekennzeichnet, dass man dem mit Freisetzungsmittel behandelten Antikörper-Reagens die zu untersuchende Urinprobe zugibt, das Gemisch Hintergrundfluoreszenzintensitätsmessungen unterwirft, einen markierten Analyten hinzufügt und die Fluoreszenzintensitätswerte des Gemisches misst.

19. Verfahren nach Anspruch 17 oder 18, dadurch gekennzeichnet, dass das mit dem Freisetzungsmittel behandelte Antikörper-Reagens einem gemäss einem der Ansprüche 1 bis 11 Reagens entspricht.

20. Verfahren zur quantitativen oder qualitativen Erfassung von 11-nor-Δ9-Tetrahydrocannabinol-9-carbonsäure in einem Fluoreszenzpolarisationsimmunoassay, dadurch gekennzeichnet, dass man der zu untersuchenden Urinprobe ein Freisetzungsmittel zufügt, das aus 1-Anilinonaphthalin-8-sulfonsäure, 1,1′-bis-(4-Anilino)naphthalin-5,5′-disulfonsäure, 2-Anilinonaphthalin-6-sulfonsäure, Lithiumdodecylsulfat, Natriumdodecylsulfat, Ammoniumdodecylsulfat, Cholsäure, Benzoesäure, Salicylsäure, 5-Chlorsalicylsäure, 5-Jodsalicylsäure, 5-Methoxysalicylsäure, 5-Sulfosalicylsäure, Witconate P 10-59 und Gemischen dieser Substanzen ausgewählt ist.

21. Verfahren nach Anspruch 20, dadurch gekennzeichnet, dass man die mit Freisetzungsmittel behandelte Urinprobe einem Antikörper-Reagens zugibt, das einen Antikörper für 11-nor-Δ9-Tetrahydrocannabinol-9-carbonsäure sowie einen wässrigen Puffer mit einem pH-Bereich von 7,0 bis 8,5 enthält, das Gemisch Hintergrundfluoreszenzintensitätsmessungen unterwirft, markierten Analyten hinzufügt und die Fluoreszenzintensitätswerte des Gemisches misst.

22. Verfahren nach Anspruch 18 oder 21, dadurch gekennzeichnet, dass die Markierung des Analyten mit Fluoresceinisothiocyanat erfolgt.

23. Verfahren nach Anspruch 20 oder 21, dadurch gekennzeichnet, dass als Freisetzungsmittel 1-Anilinonaphthalin-8-sulfonsäure, 1,1′-bis(4-Anilino)naphthalin-5,5′-disulfonsäure oder Lithiumdodecylsulfat, oder ein Gemisch von zwei oder allen drei dieser Substanzen verwendet wird.

Patentansprüche für folgenden Vertragsstaat: ES

1. Verfahren zur quantitativen oder qualitativen Erfassung von 11-nor-Δ9-Tetrahydrocannabinol-9-carbonsäure in einem Fluoreszenzpolarisationsimmunoassay, dadurch gekennzeichnet, dass man einem Antikörper-Reagens zur Verwendung beim Fluoreszenzpolarisationsimmunoassay für 11-nor-Δ9-Tetrahydrocannabinol-9-carbonsäure ein Freisetzungsmittel zufügt, das aus 1-Anilinonaphthalin-8-sulfonsäure, 1,1′-bis(4-Anilino)naphthalin-5,5′-disulfonsäure, 2-Anilinonaphthalin-6-sulfonsäure, Lithiumdodecylsulfat, Natriumdodecylsulfat, Ammoniumdodecylsulfat, Cholsäure, Benzoesäure, Salicylsäure, 5-Chlorsalicylsäure, 5-Jodsalicylsäure, 5-Methoxysalicylsäure, 5-Sulfosalicylsäure, Witconate P 10-59 und Gemischen dieser Substanzen ausgewählt ist.

2. Verfahren nach Anspruch 1, dadurch gekennzeichnet, dass man dem mit Freisetzungsmittel behandelten Antikörper-Reagens die zu untersuchende Urinprobe zugibt, das Gemisch Hintergrundfluoreszenzintensitätsmessungen unterwirft, markierten Analyten hinzufügt und die Fluoreszenzintensitätswerte des Gemisches misst.

3. Verfahren zur quantitativen oder qualitativen Erfassung von 11-nor-Δ9-Tetrahydrocannabinol-9-carbonsäure in einem Fluoreszenzpolarisationsimmunoassay, dadurch gekennzeichnet, dass man der zu untersuchenden Urinprobe ein Freisetzungsmittel zufügt, das aus 1-Anilinonaphthalin-8-sulfonsäure, 1,1′-bis-(4-Anilino)naphthalin-5,5′-disulfonsäure, 2-Anilinonaphthalin-6-sulfonsäure, Lithiumdodecylsulfat, Natriumdodecylsulfat, Ammoniumdodecylsulfat, Cholsäure, Benzoesäure, Salicylsäure, 5-Chlorsalicylsäure, 5-Jodsalicylsäure, 5-Methoxysalicylsäure, 5-Sulfosalicylsäure, Witconate P 10-59 und Gemischen dieser Substanzen ausgewählt ist.

4. Verfahren nach Anspruch 3, dadurch gekennzeichnet, dass man die mit Freisetzungsmittel behandelte Urinprobe einem Antikörper-Reagens zugibt, das einen Antikörper für 11-nor-Δ9-Tetrahydrocannabinol-9-carbonsäure sowie einen wässrigen Puffer mit einem pH-Bereich von 7,0 bis 8,5 enthält, das Gemisch Hintergrundfluoreszenzintensitätsmessungen unterwirft, markierten Analyten hinzufügt und die Fluoreszenzintensitätswerte des Gemisches misst.

5. Verfahren nach einem der Ansprüche 1 bis 4, dadurch gekennzeichnet, dass das Freisetzungsmittel 1-Anilinonaphthalin-8-sulfonsäure, 1,1′-bis(4-Anilino)naphthalin-5,5′-disulfonsäure oder Lithiumdodecylsulfat, oder ein Gemisch von zwei oder allen drei dieser Substanzen ist.

6. Verfahren nach Anspruch 5, dadurch gekennzeichnet, dass das Freisetzungsmittel 1,1-bis(4-Anilino)-naphthalin-5,5′-disulfonsäure ist.

7. Verfahren nach einem der Ansprüche 1 bis 6, dadurch gekennzeichnet, dass das Freisetzungsmittel in einer solchen Menge zugefügt wird, die eine möglichst vollständige Erfassung der in der zu untersuchenden Urinprobe vorhandenen 11-nor-Δ9-Tetrahydrocannabinol-9-carbonsäure erlaubt, allerdings nicht in einer Menge, bei der die Wirksamkeit des Antikörpers wesentlich beeinträchtigt wird.

8. Verfahren nach einem der Ansprüche 1, 2 und 4 bis 7, dadurch gekennzeichnet, dass die Menge an vorhandenem Freisetzungsmittel in Mol/l die Menge (Mol/l) an Proteinen im Reagens übersteigt.

9. Verfahren nach einem der Ansprüche 1 bis 8, dadurch gekennzeichnet, dass die Menge an zugefügtem Freisetzungsmittel diejenige ist, die eine prozentuale Erfassung von mindestens 85% der in der Urinprobe vorhandenen 11-nor-Δ9-Tetrahydrocannabinol-9-carbonsäure ermöglicht.

10. Verfahren nach Anspruch 9, dadurch gekennzeichnet, dass die Menge an zugefügtem Freisetzungsmittel eine prozentuale Erfassung von 95-100% der in der Urinprobe vorhandenen 11-nor-Δ9-Tetrahydrocannabinol-9-carbonsäure ermöglicht.

11. Verfahren nach einem der Ansprüche 1, 2 und 4 bis 10, dadurch gekennzeichnet, dass das Antikörper-Reagens zusätzlich zu den bereits genannten Bestandteilen auch noch Rinder-gamma-globulin und Natriumazid enthält.

12. Verfahren nach Anspruch 11, dadurch gekennzeichnet, dass die Menge des Rinder-gamma-globulins ca. 0,01 w/v% und die Menge des Natriumazids ca. 0,2 w/v% betragen.

13. Verfahren nach Anspruch 12, dadurch gekennzeichnet, dass das Antikörper-Reagens aus einer Ziege gewonnenes Antiserum, ca. 0,1M Phosphat-Puffer mit einem pH-Wert von ca. 8, ca. 0,01 w/v% Rinder-gamma-globulin sowie ca. 0.2 w/v% Natriumazid enthält.

14. Verfahren nach Anspruch 13, dadurch gekennzeichnet, dass das Antikörper-Reagens ein Freisetzungsmittel enthält, das 1,1′-bis(4-Anilino)naphthalin-5,5′-disulfonsäure bei einer Konzentration von ca. 0,0005 w/v% ist.

15. Verfahren nach Anspruch 2 oder 4, dadurch gekennzeichnet, dass die Markierung des Analyten mit Fluoresceinisothiocyanat erfolgt.